# EUROPÄISCHE PATENTSCHRIFT

(11) **EP 1 594 458 B1**
(45) Veröffentlichungstag und Bekanntmachung des Hinweises auf die Patenterteilung: **06.05.2009**
(21) Anmeldenummer: 04710016.9
(22) Anmeldetag: 11.02.2004
(51) Int. Cl.: A61Q 19/00, A61K 8/97

(54) **VERFAHREN ZUR HERSTELLUNG EINES KOSMETISCHEN ABRASIVUMS**
METHOD FOR PRODUCING A COSMETIC ABRASIVE
PROCEDE DE PRODUCTION D'UN ABRASIF COSMETIQUE

(30) Priorität: 12.02.2003 DE 10305959
(43) Veröffentlichungstag der Anmeldung: 16.11.2005
(73) Patentinhaber: Evonik Stockhausen GmbH, 47805 Krefeld (DE)
(72) Erfinder: PETERSEN, Olaf, 40667 Meerbusch (DE); DANIEL, Günter, 47809 Krefeld (DE); FRIEBEL, Michael, 47918 Tönisvorst (DE)
(86) Internationale Anmeldenummer: PCT/EP2004/001260
(87) Internationale Veröffentlichungsnummer: WO 2004/071483

(56) Entgegenhaltungen:
- EP-A- 1 106 173
- WO-A-92/09265
- US-A- 5 830 445

## Beschreibung

Die vorliegende Erfindung betrifft ein verbessertes Verfahren zur Herstellung eines Abrasivums.

Ein wesentlicher Bestandteil kosmetischer Reinigungs- und Behandlungsmittel ist das Reibemittel, das die Aufgabe hat, die Reinigungswirkung von waschaktiven bzw. tensidartigen Komponenten mechanisch zu unterstützen.

Im Stand der Technik sind zahlreiche anorganische und organische Materialien beschrieben, die sich in Reinigungspräparaten als mechanische Reinigungs- und Behandlungsmittel einsetzen lassen, insbesondere in Handreinigern oder in sogenannten Peeling-Cremes und Spezialreinigungs-Gelen. Sie dienen hierbei zur Entfernung der oberen, abgestorbenen Hautzellen oder von Hautverunreinigungen, beispielsweise im Gesicht oder an anderen Körperteilen.

Um das Risiko zu starker Schleifwirkung anorganischer Materialien auszuschließen, ist Holzmehl als-Schleifmittel in kosmetische Produkte eingeführt worden (Haut und Beruf, Strategien zu berufsbedingten Hauterkrankungen, H. Tronnier, Grosse-Verlag, Berlin 1989, Seiten 84/85).

Es ist jedoch seit langem bekannt, daß Holzstaub durch als Allergene wirkende Inhaltsstoffe nachteilige Wirkungen auf Haut und Atemwege haben kann. Holzinhaltsstoffe, die gesundheitliche Risiken darstellen, sind z.B. Harze, Terpene, Phenole, Gerbstoffe und Chinone.

Darüber hinaus ist Holzmehl mit einem hohen Keimgehalt behaftet und als Folge seiner weichen, faserigen Struktur treten Quellprozesse im Endprodukt auf, die zu einem unerwünschten Viskositätsanstieg und damit zu gebrauchstechnischen Nachteilen führen: Ferner müssen ca. 2 Gew.-% an aufhellenden Pigmenten, wie z.B. Titandioxid zugesetzt werden, um helle, sauber wirkende und kosmetisch akzeptable Produkte zu erhalten.

Die genannten Nachteile des Holzmehls entfallen, wenn man Reibemittel auf Basis von Polyethylen- oder Polyurethan-Pulver einsetzt. Kunststoffpulver haben jedoch den Nachteil, daß sie biologisch nicht abbaubar sind und teilweise aufgrund schwierig durchzuführender Mahlprozesse zu einer unregelmäßigen Kornverteilung führen, so daß, auch bedingt durch das geringe Schüttgewicht dieser Materialien, formulierungstechnische Schwierigkeiten bei der Herstellung kosmetischer Produkte auftreten.

Weiterhin sind im Stand der Technik natürliche Abrasivstoffe, wie z.B. gewaschene und gemahlene Schalen von Walnüssen sowie gemahlene Aprikosenkerne oder Olivenkerne bekannt, die aufgrund ihrer Härte und Korngröße für die oberflächliche Hautreinigung geeignet sind. Nachteilig hieran ist jedoch, daß selbst bei Behandlung dieser Materialien mit keimtötenden Mitteln maximale Keimzahl Werte von 200/g erreicht werden (siehe z.B. Prospekt der Firma Cosmetochem AG, Riedstraße 7, CH-6330 Cham, Schweiz).

Natürliche Abrasivstoffe haben eine schonende, sehr gut reinigende Wirkung, ohne die Haut zu zerkratzen. Sie führen jedoch, wie auch Polyurethan-Pulver zu kosmetischen Produkten, die ein dunkles, schmutziges Aussehen haben. Es ist daher erforderlich, wie bei der Verwendung von Holzmehl als Abrasivum auch aufhellende Pigmente beispielsweise Titandioxid mit ca. 2 Gew.-% zusetzen.

Ein Bleichverfahren für die Schalen von Walnüssen wird in Research and Industrie,. Volume 29, March 1984, Pages 10 bis 16 beschrieben. Durch eine Behandlung mit Natrium-Hydrogensulfit und anderen Mitteln soll das Aussehen der Schalen verbessert werden, um den Export der Walnüsse aus Indien nach Europa zu erleichtern.

In der Internationalen Anmeldung WO 99/52500 werden mild abrasive Hautreinigungsmittel beschrieben, die in Form viskoser Lotionen, Cremes oder Gele bis zu 30 Gew.-% pflanzliche Abrasivstoffe, bezogen auf die Reinigungsmittel, von z.B. Naturmehlen aus gemahlenen Kernen oder Schalen, Fruchthülsen oder Samen enthalten. Darüber hinaus können diese Hautreinigungsmittel noch bis zu 20 Gew.-% anderer anorganischer und organischer Abrasivkomponenten wie z.B. Polyethylen- oder Polyamid-Pulver enthalten. Diese kosmetischen Produkte weisen insbesondere eine hohe Stabilität gegen enzymatischen Abbau der Verdickungsmittel auf, wenn als Verdickungsmittel wasserlösliche Biopolymere aus der Gruppe der extracellulären mikrobiellen Polysaccharide wie z.B. Xanthan-Gum verwendet werden.

In der WO-A-92/09265 bzw. den aus dieser internationalen Patentanmeldung hergegangenen EP 0 559 696 B1 sowie US-A-5,830,445 werden ein Verfahren zur Herstellung von mit einem Bleichmittel, insbesondere Wasserstoffperoxid behandeltem Material in feiner Verteilung aus natürlichen Schalen und/oder Kernen beschrieben sowie die Verwendung des so gewonnenen Abrasivums in kosmetischen Produkten dargestellt.

Das in diesen Patentschriften beschriebene Verfahren zur Bleichung von Naturmehlen, das Mehle bestimmter Korngröße, vorzugsweise Walnussschalenmehl, bleicht und trocknet, wird seit vielen Jahren im großtechnischen Maßstab erfolgreich angewandt. Die hiernach hergestellten Mehle haben üblicherweise eine Keimzahl kleiner als 10² KBE/g und sind frei von pathogenen Keimen, weisen eine hellbeige Farbe auf und werden in Handwaschpasten zur industriellen Handreinigung eingesetzt. Hierbei hat sich der durch die Mehle hervorgerufene abrasive Effekt zur physikalischen Entfernung von Industrieschmutzen sich seit vielen Jahren als sehr adäquat und effektiv erwiesen.

Nachteilig an diesem Verfahren ist, was sich jedoch in der Praxis gezeigt hat, daß das Bleichen der natürlichen Kern- und Schalenmehle in wasserhaltiger Suspension durchzuführen ist, wodurch auch große Mengen an Ablaugen anfallen. Darüber hinaus werden in dem Verfahren auch Stabilisierungs- und Reduktionsmittel eingesetzt, die natürlich als zusätzliche Rohstoffe dieses Verfahren verteuern in Vergleich zu einem möglichen Verfahren, in dem auf solche Stabilisierungs- und Reduktionsmittel verzichtet werden könnte.

Zur Vermeidung solcher Ablaugen wird in der EP 1 136 063 A2 ein Bleichverfahren vorgeschlagen, in dem in einem "trockenen" Prozess Persäuren auf das natürliche Mehl, das als biologisches Material aus einer Vielzahl pflanzlicher Materialien gewonnen werden kann, aufgesprüht werden und eine Aufhellung bewirken sollen. Die Mischung dieses biologischen Materials mit dem Bleichmittel soll höchstens 60 Gew.-% Wasser enthalten, wobei nach dem Mischungsvorgang ein Nachreifungsprozeß einsetzt. Nach 10 Tagen soll das während des Verfahrens gebildete Peroxid im erhaltenen Produkt nicht mehr nachweisbar sein.

Nachteilig an diesem Verfahren ist allerdings, daß die eingebrachten Chemikalien bzw. deren Reaktions- und/oder Folgeprodukte, insbesondere Restmengen an Carbonsäuren im fertigen Mehl verbleiben können, da auf entsprechende Reinigungs- bzw. Waschprozesse verzichtet wird. Durch die Bildung von Salzfrachten bei der Herstellung der kosmetischen Endprodukte, insbesondere in Handwaschpasten wirken diese Reaktions- und/oder Folgeprodukte umweltbelastend. Darüber hinaus werden auch in diesem Verfahren bis zu 5 Gew.-%, bezogen auf die das biologische Material und das Bleichmittel umfassende Mischung, Stabilisierungsmittel in Form von Moderatoren eingesetzt. Entsprechendes gilt für den Einsatz von Reduktionsmitteln zum Zerstören von überschüssigen Peroxiden.

Die DE 101 14 341 C2 beschreibt ein Verfahren zum Vermahlen und Bleichen von zu Partikeln zerkleinertem natürlichem cellulosehaltigem Material mit einem Bleichmittel wie Wasserstoffperoxid, in dem die Partikel des Materials mit dem Bleichmittel versetzt und während der Einwirkung des Bleichmittels bei einem Feststoffgehalt von mehr als 60% in einer mit hoher Geschwindigkeit umlaufenden Mahlorgane aufweisenden Schlag- oder Schleudermühle vermahlen werden. Dem Bleichmittel können unter 3 % Alkalien oder Natriumcarbonat (Soda) sowie weitere Hilfsmittel wie Tetraacetylethylendiamin (TAED) oder Peressigsäure zugesetzt sein. Aufgrund des hohen Feststoffgehaltes des Mahlgutes handelt es sich hierbei wie auch bei dem in der EP 1 136 063 A2 beschriebenen Bleichverfahren um einen "trockenen" Prozess, der die vorgenannten Nachteile aufweist, nämlich die eingebrachten Chemikalien bzw. deren Reaktions-und/oder Folgeprodukte können im fertigen Mehl verbleiben, da auf entsprechende Reinigungs- bzw. Waschprozesse nach der Bleichung verzichtet wird.

Die EP 1 106 173 A2 beschreibt ein einstufiges Verfahren zum Bleichen von Naturstoffen, insbesondere Maiskolbenmehl bei der Herstellung von Abrasiva für kosmetische Zwecke, wobei Wasser, das zu bleichende Naturstoffmehl und gegebenenfalls andere Hilfsstoffe gemischt werden, Wasserstoffperoxid und Alkalihydroxid der Mischung zugesetzt und bei Absenkung des pH-Wertes der Mischung eine Säure zugegeben wird, bis das Wasserstoffperoxid nicht mehr nachzuweisen ist, und dann andere Hilfsstoffe der Mischung zugesetzt werden. Nachteilig an diesem Verfahren ist auch hier, daß die eingebrachten Chemikalien bzw. deren Reaktions- und/oder Folgeprodukte im fertigen Mehl verbleiben können, da auf entsprechende Reinigungs- bzw. Waschprozesse explizit verzichtet wird.

Aus dem geschilderten Stand der Technik wird deutlich, daß noch immer ein dringender Bedarf an einem Herstellungsverfahren für Abrasiva besteht, das es erlaubt, den Produktionsprozess der mikrobiologischen Dekontaminierung, der Deodorierung und des Bleichens von natürlichen Kern- und Schalenmehlen mit Bleichmitteln umweltschonender und wirtschaftlicher zu gestalten, insbesondere im Hinblick auf eine Erhöhung der Ausbeute, Verringerung und/oder Eliminierung von Chemikalien, Reduzierung der Ablaugenmengen sowie einer Verkürzung der Prozesszeit.

Aufgabe war es daher, ein solches Verfahren zu finden, dessen Bleichergebnis im Hinblick auf Keimgehalt, Geruch und Farbe nicht nur zu Abrasiva führt, die in ihrem Eigenschaftsprofil vergleichbar bzw. besser sind als die nach dem in der EP 0 559 696 B1 beschriebenen Verfahren erhaltenen Abrasiva, die gegenwärtig bei ihrem Einsatz in kosmetischen Reinigungsmitteln bei geringstmöglicher Konzentration bzw. unter völligem Verzicht an aufhellenden Substanzen optisch helle und kosmetisch akzeptable Produkte liefern, sondern auch vollständig auf den Einsatz von Stabilisatoren und Reduktionsmitteln im Produktionsprozeß verzichtet und die nach dem Waschprozess des mikrobiologisch dekontaminierten, deodorierten und gebleichten Mehles anfallenden Abwässer bezüglich der biologischen Abbaubarkeit verbessert und insbesondere den gesamten Produktionsprozeß in betriebswirtschaftlicher Hinsicht kostengünstiger gestaltet.

Überraschend wurde gefunden, daß die obige Aufgabe durch ein Verfahren zur Herstellung eines abrasiven Stoffes gelöst werden kann, bei dem vollständig auf den Einsatz von Stabilisatoren und Reduktionsmitteln im Produktionsprozeß verzichtet werden kann. Hierbei werden natürliche Kerne, Schalen, Fruchthülsen und/oder Samen zu einem Mehl definierter Korngröße gemahlen, anschließend das erhaltene Mehl in wässriger Suspension mit 1,0 bis 10,0 Gew.-% eines Bleichmittels, bezogen auf die gesamte Ansatzmenge, behandelt, wobei das erfindungsgemäßen Verfahren dadurch gekennzeichnet ist, daß die Zugabe des Bleichmittels in zwei Schritten erfolgt, wobei im ersten Schritt nach Zugabe von 20 bis 40 Gew.-% des Bleichmittels, bezogen auf die gesamte Einsatzmenge des Bleichmittels, ein pH-Wert-Bereich von 3 bis 5 erhalten wird und die Zugabe von 60 bis 80 Gew.-% des Bleichmittels im zweiten Schritt gemeinsam kontinuierlich mit der Zugabe einer Alkalienlösung im pH-Wert-Bereich von 5 bis 11 erfolgt.

Die Starttemperatur zur Behandlung der wässrigen Suspension des Mehles aus natürlichen Kernen, Schalen, Fruchthülsen und/oder Samen liegt bei 20 bis 40°C, vorzugsweise 25 bis 35°C und besonders bevorzugt 28 bis 32°C.

Erfindungsgemäß werden als natürliche Schalen- oder Kernmehle Walnußschalenmehl, Mandelschalenmehl, Haselnußschalenmehl, Olivenkernmehl, Aprikosenkernmehl, Pfirsichkernmehl, Kirschkernmehl, oder sonstiges natürliches Schalen- oder Kernmehl, beispielsweise von Palmkernen und Kokosnüssen, Jojobafrüchten, Macademia-Nüssen und anderen Nüssen, Pistazien- und Pinienschalen und anderes Kernobst sowie ein beliebiges Gemisch der genannten Materialien eingesetzt. Besonders bevorzugt als natürliches Schalen- oder Kernmehl ist erfindungsgemäß Walnußschalenmehl.

Darüber hinaus lassen sich auch weitere im Stand der Technik als milde Abrasiva bekannte Pflanzenmehle aus Fruchthülsen und Samen, wie z.B. Maiskolbenmehl, Weizenkleie, Hafermehl aber auch beliebige Holzmehle mit dem erfindungsgemäßen Verfahren bleichen.

Um eine definierte Korngröße der im erfindungsgemäßen Verfahren einzusetzenden Mehle von natürlichen Kernen, Schalen, Fruchthülsen und/oder Samen zu erhalten, werden diese in an sich bekannter Weise zu einem Mehl, gegebenenfalls unter Einbeziehung einer Klassifizierung durch Siebe gemahlen. Mehle, die eine Korngröße von 50 bis 20 000 µm, vorzugsweise von 70 bis 1000 µm und besonders bevorzugt von 80 bis 400 µm besitzen, können im erfindungsgemäßen Verfahren verwendet werden.

Zum Mahlen der Mehle können die im Stand der Technik bekannten Zerkleinerungsapparate bzw. Mühlen eingesetzt werden, wie sie z. B. in der EP 0 559 696 ausgeführt worden sind, insbesondere Feinprallmühlen mit Pendel- oder Plattenschlägerwerk, Passagenwalzwerke, Hammerschlag- oder Stiftmühlen gegebenenfalls mit Klassierungsaggregaten, wie z. B. Condux-Mühlen.

Die zu einem Mehl definierter Korngröße gemahlenen natürlichen Kerne, Schalen, Fruchthülsen und/oder Samen werden in wässriger Suspension mit einem Bleichmittel behandelt. Als Bleichmittel können alle Verbindungen verwendet werden, die eine irreversible Zerstörung der Chromophore in diesen Naturmehlen gewährleisten, wobei die gebleichten Mehle bei der erfindungsgemäßen Bleichbehandlung nicht oder nur unwesentlich chemisch verändert werden, so daß sie als Abrasiva in kosmetischen Produkten Anwendung finden können. Solche Bleichmittel sind z.B. sogenannte oxidierende Bleichmittel, wie sie beispielsweise in Ullmanns Encyklopädie der technischen Chemie, 4. Auflage, Bd. 8, Seiten 589 bis 595 beschrieben sind. Bevorzugt sind anorganische und organische Peroxide wie z.B. Wasserstoffperoxid, Natriumperoxid, Bariumperoxid oder Peroxycarbonsäuren, insbesondere Peroxyameisensäure, Peroxyessig- und Peroxypropionsäure, die für den Fachmann in bekannter Weise auch in situ hergestellt bzw. erfindungsgemäß eingesetzt werden können. Die vorgenannten Verbindungen können allein aber auch als Mischung von wenigstens zwei dieser Verbindung im erfindungsgemäßen Verfahren Anwendung finden. Erfindungsgemäß bevorzugtes Bleichmittel ist wäßrige Wasserstoffperoxidlösung, das in einer Konzentration von 1,0 Gew.-% bis 10,0 Gew.-%, vorzugsweise 1,0 Gew.-% bis 3,0 Gew.-%, bezogen auf die gesamte Ansatzmenge, dem Bleichgut zugesetzt wird.

Nach der vorliegenden Erfindung ist es zwingend erforderlich, daß die Zugabe des Bleichmittels in zwei Schritten erfolgt, wobei im ersten Schritt nach Zugabe von 20 bis 40 Gew.-% des Bleichmittels, vorzugsweise 25 bis 35 Gew.-%, bezogen auf die gesamte Einsatzmenge des Bleichmittels, ein pH-Wert-Bereich von 3 bis 5 erhalten wird. Vorzugsweise liegt der pH-Wert der wässrigen Suspension des Mehles aus natürlichen Kernen, Schalen, Fruchthülsen und/oder Samen nach Zugabe von einem Drittel der Gesamtmenge des benötigten Bleichmittels bei 4 bis 5. Anschließend kann zur Verbesserung des Bleichergebnisses die erhaltene Suspension über einen Zeitraum von ca. 60 bis 180 Minuten gerührt werden. Dies ist jedoch für den Erfolg des erfindungsgemäßen Verfahrens nicht zwingend erforderlich.

Die Zugabe von 60 bis 80 Gew.-%, vorzugsweise 65 bis 75 Gew.-% des Bleichmittels im zweiten Schritt erfolgt kontinuierlich gemeinsam mit der Zugabe einer Alkalienlösung im pH-Wert-Bereich von 5 bis 11, wobei bei Verbrauch der eingesetzten Bleichmittelmenge auch die Zugabe der Alkalienmenge beendet ist.

Die Zugabe der benötigten Bleichmittelmenge zum Bleichgut erfolgt in beiden Schritten aus Gründen der besseren Dosierung bzw. der pH-Wert-Steuerung vorzugsweise in flüssiger Form. Diese Zugabeform des Bleichmittels ist hierauf jedoch nicht beschränkt, sondern ist abhängig von der Wahl des Bleichgutes bzw. des einzusetzenden Bleichmittels. So ist es erfindungsgemäß auch möglich, die Zugabe des Bleichmittels zunächst in fester Form zur Bleichgutsuspension zu geben und im zweiten Schritt das Bleichmittel in flüssiger Form kontinuierlich gemeinsam mit der Alkalienlösung zuzudosieren.

Da es sich bei dem erfindungsgemäßen Verfahren um einen industriellen Großprozeß handelt, ist aus Gründen der Wirtschaftlichkeit Wasser das besonders bevorzugte Lösungsmittel des benötigten Bleichmittels. Nichtsdestoweniger kann es auch hier im Hinblick auf die Art des Bleichgutes bzw. des benötigten Bleichmittels angezeigt sein, das Bleichmittel in einem üblichen organischen Lösungsmittel zu lösen, um die gewünschte Bleichwirkung zu erzielen. Die erhaltene Bleichmittellösung wird dann, wie beschriebenen, in zwei Schritten der Suspension des Bleichgutes zugesetzt.

Die kontinuierliche Zuführung der restlichen Bleichmittelmenge mit der Alkalienzugabe erfolgt über einen Zeitraum von 95 bis 115, vorzugsweise 100 bis 110 Minuten.

Als Alkalienlösung werden üblicherweise wässrige Lösungen von Hydroxiden der Alkalimetalle, insbesondere von Natrium- und Kaliumhydroxid eingesetzt. Weiterhin sind auch Ammoniumhydroxid und die Hydroxide der Erdalkalimetalle sowie auch die Carbonate der Alkalimetalle, insbesondere Natriumcarbonat und Kaliumcarbonat verwendbar. Besonders bevorzugt ist jedoch wässrige Natriumhydroxidlösung bzw. Natronlauge als erfindungsgemäß einzusetzende Alkalienlösung, insbesondere als 35 bis 75 %-ige, vorzugsweise 45 bis 60 %-ige und besonders bevorzugt als 50 %-ige wässrige Lösung.

Nach Zugabe der Teilbleichmittelmenge im ersten Verfahrensschritt beginnt die Dosierung von einem Teil der benötigten Alkalienmenge bzw. erfolgt die kontinuierliche simultane Zugabe der Bleichmittelrestmenge bis ein pH-Wert von höchstens 11 erreicht, wobei dieser maximale pH-Wert 5 bis 25, vorzugsweise 10 bis 20 und besonders bevorzugt 15 Minuten auf diesem Wert gehalten wird.

Danach erfolgt die Dosierung der Natronlauge bzw. des Wasserstoffperoxides in der Weise, daß der pH-Wert der Suspension nach dem Halten auf dem gewählten Maximalwert auf pH-Wert 8,0 abgesenkt und dort innerhalb einer Schwankungsbreite von höchstens ± 0,2 gehalten wird.

In einer Ausführungsform des erfindungsgemäßen Verfahren wird das Verfahren so geführt, daß die gemeinsame kontinuierliche Zugabe von Bleichmittel und Alkalienlösung in die Suspension bei Erreichen eines pH-Wertes von höchstens 11 unterbrochen und nach Absenken des pH-Wertes der Suspension auf pH-Wert 8,0 wiederaufgenommen wird.

In einer bevorzugten Ausführungsform des erfindungsgemäßen Verfahrens wird das Absenken des pH-Wertes auf pH 8,0 in der Weise erreicht, daß lediglich die Zugabe der Alkalienlösung unterbrochen wird, während der Bleichgutsuspension kontinuierlich Bleichmittel zugegeben wird bis zum Erreichen des Haltewertes von pH 8,0. Bei Erreichen dieses Wertes wird dann die gemeinsame Zugabe von Bleichmittel und Alkalienlösung wiederaufgenommen und zwar dergestalt, daß pH 8,0 innerhalb einer Schwankungsbreite von höchstens ± 0,2 gehalten wird.

Üblicherweise ist bei Verbrauch des Bleichmittels auch gleichzeitig die Zugabe an Alkalienlösung zu beenden.

Die Verfahrensdauer des erfindungsgemäßen Verfahrens, die sich aus Bleichung, Nachbleichung, Chargierzeit und Ablasszeit zusammensetzt beträgt 160 Minuten.

Aufgrund dieser schonenden Verfahrensführung liegt die Endtemperatur der natürlichen Kern-, Schalen-, Fruchthülsen- und/oder Samenmehl-Suspension nach der Bleichmittelbehandlung, insbesondere mit Wasserstoffperoxid-Lösung lediglich ca. 15 bis 20°C über der Starttemperatur der Bleichbehandlung.

Die nach dem erfindungsgemäßen Verfahren behandelten Mehle von natürlichen Kernen, Schalen, Fruchthülsen und/oder Samen werden nun durch übliche Filtrationsmethoden abgetrennt. Besonders vorteilhaft ist die Abtrennung mittels einer Siebschneckenzentrifuge. Das separierte Mehl wird hierbei kontinuierlich gewaschen, wobei die Waschwassertemperatur 60 bis 95° C, vorzugsweise 80 bis 90° C beträgt. Ausgeschleuste Feinstanteile des Gutproduktes können vorteilhaft in den Separationsvorgang zurückgeführt werden, so daß im abgeschlagenen Waschwasser keinerlei Feststoffe, insbesondere kein Gutprodukt mehr ausgetragen werden.
Dieses anfallende Abwasser ist lediglich gering belastet, so daß es möglich ist, dieses Abwasser über das städtische Kanalnetz zu entsorgen.

Der vorstehend beschriebene erfindungsgemäße Filtrations- und Abtrennungsprozeß ist besonders wirtschaftlich, weil auf den herkömmlichen Einsatz von zwei Dekantem verzichtet werden kann. Hierbei muß nämlich nach der ersten Abtrennung des Gutproduktes mittels des ersten Dekanters das erhaltene feuchte Mehl wiederum mit heißem Wasser (ca. 80°C) verdünnt werden, das dann anschließend bei einer Mischtemperatur von ca. 60 bis 65°C mittels des zweiten Dekanters vom Waschwasser befreit und danach getrocknet wird. Demgegenüber führt die erfindungsgemäße Verfahrensführung zu einer Verringerung der anfallenden Waschwassermengen sowie zur Verringerung an Produktausbeute.

Die anschließende Trocknung des Mehls erfolgt vorzugsweise durch Wirbelschichttrocknung des Mehls. Hierdurch kann eine das Produkt schonende Verfahrensweise gewählt werden, in dem beispielsweise mittels Warmluft bei Temperaturen von 30 bis 60°C, vorzugsweise von 35 bis 55°C und besonders bevorzugt von 40 bis 45°C das Produkt getrocknet wird. Innerhalb der Wirbelschicht wird dann in einer zweiten Zone das Trocknungsgut mit nachgeschalteter Kaltluft gekühlt und gelangt zur Abfüllungsvorrichtung, in der das Produkt in geeignete Behältnisse, wie z.B. Säcke, BigBags oder Container abgefüllt wird.

Das nach dem erfindungsgemäßen Verfahren erhaltene Mehl weist dann im so getrockneten Zustand eine Restfeuchte von 8,0 ± 1,0 Gew.-% auf und ist mikrobiologisch einwandfrei. Die Menge der Restfeuchtigkeit entspricht hierbei der Restfeuchtigkeit des Ausgangsmaterials.

Das erfindungsgemäße Verfahrensprodukt ist sofort als Abrasivum in kosmetischen Präparaten beispielsweise in lösungsmittelfreien oder lösungsmittelhaltigen Handwaschpasten, in wasserfreien Hautreinigungsmitteln und in Peeling-Cremes verwendbar, wobei die Verwendung in Hautreinigungsmitteln besonders bevorzugt ist, und benötigt nicht, wie das nach dem im Stand der Technik beschriebene Verfahren in der DE 100 08 816 A1 bzw. EP 1 136 063 A2 erhaltene Abrasivum einen 24-stündigen Nachreifungsprozess.

Das erfindungsgemäße Verfahrensprodukt kann bei Verwendung von Wasserstoffperoxid als Bleichmittel Restmengen an Wasserstoffperoxid von 0 bis maximal 250 ppm aufweisen. Diese Restmenge wirkt anfangs mikrobiell schützend auf das gebleichte Mehl und hat sich innerhalb weniger Tage zersetzt. Bei Einsatz des Mehls innerhalb dieser Zeit wirken sich diese Restmengen an Wasserstoffperoxid sich nicht nachteilig auf die kosmetische Formulierung aus.

Das erfindungsgemäße Verfahren erlaubt es nicht nur, den Produktionsprozess der mikrobiologischen Dekontaminierung, der Deodorierung und des Bleichens von natürlichen Kern- und Schalenmehlen mit Bleichmitteln umweltschonender und wirtschaftlicher zu gestalten, insbesondere im Hinblick auf eine Erhöhung der Ausbeute, Verringerung und/oder Eliminierung von Chemikalien, Reduzierung der Ablaugenmengen sowie einer Verkürzung der Prozesszeit, sondern es konnte überraschenderweise auch festgestellt werden, daß die erhaltene Verfahrensprodukte nicht nur eine vergleichbare, sondern zum Teil sogar verbesserte Produktqualität zu im Stand der Technik bekannten Abrasiva aufweisen, insbesondere im Hinblick auf die Helligkeit der Produkte. So konnte mittels physikalischer Farbmessung mit einem Farbmeßgerät nach DIN 5033 gefunden werden, daß die erfindungsgemäßen Verfahrensendprodukte einen L*-Wert von mindestens 79,5 aufweisen.

Die Werteskala der Werte, die der L*-Wert annehmen kann, erstreckt sich von 0 für ideal schwarze Farben bis 100 für Ideal-Weiß (siehe DIN 5033 sowie dort zitierte DIN-Normen; vgl. auch DIN 6174). Besonderes vorteilhaft lassen sich nach dem erfindungsgemäßen Verfahren Produkte erhalten, deren L*-Wert mindestens 81,0, vorzugsweise 82,5 beträgt, wobei Verfahrensprodukte mit einem L*-Wert von mindestens 85,0 besonders bevorzugt sind.

Die Vorteile des erfindungsgemäßen Verfahrens sind im einzelnen:
Es kann im Gegensatz zu den in der EP 0 559 696 B1 sowie DE 100 08 816 A1 bzw. EP 1 136 063 A2 beschriebenen Verfahren zur Bleichung von Naturmehlen im Produktionsprozess komplett auf den Einsatz von Stabilisatoren und Reduktionsmittel verzichtet werden. Insbesondere ist es möglich, den vollständigen Verfahrensablauf mittels pH-Elektroden zu steuern.

Im Vergleich zu dem in der EP 0 559 696 B1 beschriebenen Verfahren konnten die Einsatzmengen an zur Bleichung benötigtem Wasserstoffperoxid reduziert werden. Insbesondere die erfindungsgemäßen Verfahrensschritte, bei denen die gemeinsame kontinuierliche Zugabe von Bleichmittel und Alkalienlösung in die Suspension bei Erreichen eines pH-Wertes von höchstens 11, Halten des pH-Wertes und nach Absenken des pH-Wertes der Suspension auf pH-Wert 8 wiederaufgenommen wird, wobei bei Verbrauch des Bleichmittels auch gleichzeitig die Zugabe an Alkalienlösung zu beenden ist, führte zu einer Reduzierung der Bleichmittelmenge um mehr als 25% im Vergleich zu dem im EP 0 559 696 B1 beschriebenen Verfahren. Gleiches gilt für die Menge an Alkalienlösung, insbesondere die Natronlaugenmenge konnte um bis zu 75% reduziert werden. Dies führte gleichzeitig auch zu einer signifikanten Reduktion des Ausbeuteverlustes, der aufgrund dieser Verfahrenführung während der Behandlung mit Wasserstoffperoxid-Lösung und z.B. Natronlauge von ca. 8,0 auf ca. 2,5% verringert werden konnte.

Das erfindungsgemäße Verfahren ermöglicht eine das Produkt schonende Verfahrensführung, da die Endtemperatur der Walnussschalenmehl-Suspension nach der Behandlung mit Wasserstoffperoxid-Lösung als Bleichmittel lediglich ca. 15 bis 20°C über der Starttemperatur der Bleichbehandlung liegt. Hierdurch wird eine Reduzierung der Belastung des Waschwassers und damit auch eine Verringerung der Ablaugen- bzw. Waschwassermenge selbst erreicht.

Dies zeigt sich daran, daß der chemische Sauerstoffbedarf (CSB-Wert) zum Abbau des Waschwassers, das nach dem in der beschriebenen EP 0 559 696 B1 Verfahren erhalten wird, von ca. 40.000 bis 60.000 mg mit dem erfindungsgemäßen Verfahren auf ca. 10.000 - 15.000 mg O₂/I reduziert werden konnte. Die Abbaurate liegt nach 5 Tagen (BSB₅-Wert) hierbei bei 27-38% (Einwaage: 10-50 ml/l).

Mit Hilfe des Beispiels 1 wird das Verfahren zur Herstellung des erfindungsgemäßen abrasiven Stoffes näher erläutert.

### Beispiel 1

### Bleichprozeß von natürlichem Schalen- oder Kernmehl mit Wasserstoffperoxid

Der Bleichprozess wird in einem 500-Liter Reaktionsbehälter aus Edelstahl mit einem Intensivrührwerk und integrierter pH-Wert- sowie Temperaturmessung durchgeführt. Die Ansatzgröße beträgt 330,9 kg.

### Rezeptur zu Beispiel 1

| **Rohstoff** | **Einsatzmenge/kg** |
|---|---|
| Wasser | 215 bis 220 |
| Schalen- oder Kernmehl Korngröße kleiner 200µm | 100 bis 105 |
| Wasserstoffperoxid 35%ig | 7,3 bis 7,6 |
| Natronlauge 50%ig | 2,2 bis 2,3 |
| Wasser zum Waschen | 135 bis 150 |

Das Wasser wird in einem Reaktionsbehälter vorgelegt und die o.g. Einsatzmenge an Walnussschalenmehl sowie ein Drittel der Wasserstoffperoxid-Gesamtmenge eingetragen, wobei zu gewährleisten ist, daß sich das Walnußschalenmehl in der Suspension nicht absetzen kann. Die Starttemperatur beträgt 30°C. Der pH-Wert dieser Suspension liegt zwischen 4 und 5.

Anschließend beginnt die Dosierung von einem Teil der 50%igen Natronlaugenlösung bzw. erfolgt die simultane Bleichmittelzugabe entsprechend Zweidrittel der o.g. Wasserstoffperoxid-Gesamtmenge bis ein pH-Wert von 10 erreicht und 15 Minuten auf diesem Wert gehalten wird.

Danach erfolgt die Dosierung der Natronlauge bzw. des Wasserstoffperoxides in der Weise, daß der pH-Wert der Suspension nach dem Halten auf dem gewählten Maximalwert auf pH-Wert 8,0 abgesenkt und dort gehalten wird.

Während der Reaktion muss eine stetige Durchmischung der Suspension erfolgen, um das Absetzen des Walnussschalenmehls zu vermeiden.
Ein weiterer zweiter Pufferbehälter gleicher Dimension und ebenfalls mit Rührorgan ist notwendig, in den das fertig gebleichte Mehl gepumpt werden kann.

Nach 105 Minuten reiner Bleichzeit wird die Natronlaugezugabe unterbrochen und die erhaltene Suspension in einen Pufferbehälter abgepumpt, der ebenfalls ein Intensivrührwerk aufweist, um ein Absetzen des Walnußschalenmehls zu vermeiden. Die Temperatur des fertiggebleichten Ansatzes liegt bei ca. 20°C im Pufferbehälter über der Starttemperatur des Rohansatzes.

Mittels einer Siebschneckenzentrifuge wird das Ansatzwasser abgetrennt und das gebleichte Mehl kontinuierlich mit 80°C heißem Wasser gewaschen.

Der in der Zentrifuge gewaschene, abgeschleuderte Feststoff wird in einen Wirbelschichttrockner kontinuierlich eingetragen und nach der Trocknung mittels warmer, nachfolgend kalter Luft auf eine Restfeuchte von 7,0 bis 9,0 % einem Abfüllsystem zugeführt.

In den Beispielen 2 und 4 werden eine lösungsmittelfreie und eine lösungsmittelhaltige erfindungsgemäße Formulierung einer Handwaschpaste und eines wasserfreien Hautreinigungsmittels mit gebleichtem Schalen- und/oder Kernmehl angegeben.

### Beispiel 2

| **Formulierung einer lösungsmittelfreien Handwaschpaste** | |
|---|---|
| **Rohstoff** | **Einsatzmenge/Gew.-%** |
| Tensidkombination bestehend aus Natriumlaurylethersulfat 28%ig und Cocoamidopropylbetain 30%ig | 47,3 |
| Rizinusölsulfonat 68%ig | 10,6 |
| Wasser | 23,88 |
| Carboxymethylcellulose | 1,0 |
| Heteropolysaccharid | 0,3 |
| Gebleichtes Schalen- oder Kernmehl | 15,0 |
| Olein | 1,0 |
| Titandioxid | 0,5 |
| Zitronensäure | 0,14 |
| Konservierungsmittel | 0,08 |
| Parfüm | 0,2 |

### Beispiel 3

| **Formulierung einer lösungsmittelhaltigen Handwaschpaste** | |
|---|---|
| **Rohstoff** | **Einsatzmenge/Gew.-%** |
| Tensidkombination bestehend aus Natriumlaurylethersulfat 28%ig und Cocoamidopropylbetain 30%ig | 51,28 |
| Rizinusölsulfonat 68%ig | 9,8 |
| Wasser | 2,7 |
| Carboxymethylcellulose | 1,05 |
| Organophiler Bentonit | 1,7 |
| Isohexadecan | 17,5 |
| Gebleichtes Walnussschalenmehl | 13,0 |
| Siedesalz | 2,0 |
| Titandioxid | 0,5 |
| Konservierungsmittel 30%ig | 0,27 |
| Parfüm | 0,2 |

### Beispiel 4

| **Formulierung eines wasserfreien Hautreinigungsmittels** | |
|---|---|
| **Rohstoff** | **Einsatzmenge/Gew.-%** |
| Tensid: Fettalkohol C12-C18, 5EO | 19,5 |
| Lösungsmittel bestehend aus Dimethyladipat, -glutarat, -succinat | 50,0 |
| Vernetztes Polyacrylsäure-Natriumsalz | 4,0 |
| Celluloseacetobutyrat | 3,5 |
| Isooctylstearat | 3,9 |
| Gebleichtes Olivenkemmehl | 13,0 |
| Organophiler Bentonit | 4,3 |
| Titandioxid | 1,2 |
| Pyrogene Kieselsäure | 0,3 |
| Parfüm | 0,3 |

Die Herstellung der Produkte erfolgt nach den üblichen bekannten Verfahren, die allgemein für die Formulierung von Tensid-Systemen bekannt sind. (G. Ziolkowski, Kosmetik-Jahrbuch 1986, 1987, 1989, Verlag für Chemische Industrie, H. Ziolkowski KG, Augsburg, Kosmetik Georg-Thieme-Verlag Stuttgart).

### Physikalische Farbmessung des Helligkeitsgrades der Verfahrensprodukte (L*-Wert) mit einem Farbmeßgerät nach DIN 5033

Zur Messung des Helligkeitsgrades wurde als Meßprinzip die Farb- und Farbdifferenzmessung nach dem 3-Bereichsverfahren gemäß DIN 5033 verwendet.

Als Meßgerät hierfür diente das 3-Bereichs-Farbmeßgerät MIKRO COLOR II der Fa. Dr. Lange Bruno Lange GmbH Berlin Industriemeßtechnik, Düsseldorf mit einem optischen Aufbau gemäß DIN 5033. Als Lichtquelle wurde eine Xenon-Blitz-Lampe verwendet, die in Verbindung mit einer Ulbricht`schen Kugel zur diffusen Beleuchtung der zu messenden Probe dient - Normlichtart D65. Gemessen wird hierbei nach DIN 5033 die diffuse Reflexion der Probe unter einem Winkel von 8°.
Für die Farbmessung gemäß DIN 5033 wurde als Bezugsstandard bzw. als Weißstandard der nach DIN 55350 Teil 18, 4.1.2 zertifizierte Kalibrierstandard LZM 076 verwendet:

| | |
|---|---|
| Standardnummer: | 010799 |
| | |
| Normfarbwert X: | 74,5 |
| Normfarbwert Y: | 79,5 |
| Normfarbwert Z: | 83,0 |
| Normlichtart: | D65 |
| Normalbeobachter: | 10° |
| Meßgeometrie: | d/8° |

In der nachfolgenden Tabelle werden die L*-Werte verschiedener Chargen von erfindungsgemäß gebleichtem Walnußschalenmehl angegeben.

| Datum | Chargennummer | L*-Wert | |
|---|---|---|---|
| 07.08.2001 | 40311305 | 79,0 | entsprechend EP 0 559 696 B1 |
| 20.02.2002 | 40326684 | 80,0 | entsprechend EP 0 559 696 B1 |
| 11.11.2002 | 40347877 | 83,0 | erfindungsgemäß |
| 09.05.2003 | 40360181 | 81,9 | erfindungsgemäß |
| 12.05.2003 | 40360184 | 82,7 | erfindungsgemäß |
| 13.05.2003 | 40360185 | 85,5 | erfindungsgemäß |
| 14.05.2003 | 40360186 | 82,8 | erfindungsgemäß |
| 15.05.2003 | 40361972 | 81,9 | erfindungsgemäß |
| 16.05.2003 | 40361973 | 81,9 | erfindungsgemäß |
| 20.05.2003 | 40361618 | 79.9 | erfindungsgemäß |
| 21.05.2003 | 40361619 | 81,5 | erfindungsgemäß |
| 22.05.2003 | 40361620 | 80,8 | erfindungsgemäß |
| 23.05.2003 | 40361621 | 82,3 | erfindungsgemäß |
| 26.05.2003 | 40361622 | 80,9 | erfindungsgemäß |
| 27.05.2003 | 40361623 | 82,2 | erfindungsgemäß |
| 28.05.2003 | 40361624 | 82,5 | erfindungsgemäß |
| 29.05.2003 | 40361625 | 82,3 | erfindungsgemäß |
| 30.05.2003 | 40361626 | 85,0 | erfindungsgemäß |
| 31.05.2003 | 40361627 | 85,4 | erfindungsgemäß |
| 01.06.2003 | 40363248 | 86,1 | erfindungsgemäß |
| 02.06.2003 | 40363249 | 85,2 | erfindungsgemäß |
| 03.06.2003 | 40363250 | 86,6 | erfindungsgemäß |
| 04.06.2003 | 40363251 | 86,7 | erfindungsgemäß |

## Patentansprüche

1. Verfahren zur Herstellung eines abrasiven Stoffes, wobei natürliche Kerne, Schalen, Fruchthülsen und/oder Samen zu einem Mehl definierter Korngröße gemahlen werden, das Mehl in wässriger Suspension mit 1,0 bis 10,0 Gew.-% eines Bleichmittels, bezogen auf die gesamte Ansatzmenge, behandelt wird, **dadurch gekennzeichnet, daß** die Zugabe des Bleichmittels in zwei Schritten erfolgt, wobei im ersten Schritt nach Zugabe von 20 bis 40 Gew.-% des Bleichmittels, bezogen auf die Gesamtmenge ein pH-Wert-Bereich von 3 bis 5 erhalten wird und die Zugabe von 60 bis 80 Gew.-% des Bleichmittels im zweiten Schritt gemeinsam kontinuierlich mit der Zugabe einer Alkalienlösung im pH-Wert-Bereich von 5 bis 11 erfolgt.

2. Verfahren nach Anspruch 1, **dadurch gekennzeichnet, daß** die gemeinsame kontinuierliche Zugabe von Bleichmittel und der Alkalienlösung in die Suspension bei Erreichen eines pH-Wertes von höchstens 11 unterbrochen und nach Absenken des pH-Wertes auf pH-Wert 8,0 wiederaufgenommen wird.

3. Verfahren nach Anspruch 2, **dadurch gekennzeichnet, daß** die Unterbrechung der gemeinsamen kontinuierlichen Bleichmittel- und Alkalienlösungzugabe durch Unterbrechung der Alkalienlösungzugabe erreicht wird.

4. Verfahren nach den Ansprüchen 1 bis 3, **dadurch gekennzeichnet, daß** bei Erreichen eines pH-Wertes von höchstens 11 dieser maximale pH-Wert 5 bis 25 Minuten auf diesem Wert gehalten wird.

5. Verfahren nach den Ansprüchen 1 bis 4, **dadurch gekennzeichnet, daß** nach Wiederaufnahme der gemeinsamen Zugabe von Bleichmittel und Alkalienlösung die Zugabe in der Weise erfolgt, so daß ein pH-Wert 8,0 bis zum Ende der gemeinsamen Zugabe innerhalb einer Schwankungsbreite von ± 0,2 eingehalten wird.

6. Verfahren nach den Ansprüchen 1 bis 5, **dadurch gekennzeichnet, daß** bei Verbrauch der eingesetzten Bleichmittelmenge auch die Zugabe der Alkalienmenge beendet wird.

7. Verfahren nach den Ansprüchen 1 bis 6, **dadurch gekennzeichnet, daß** als natürliche Kern-, Schalen-, Fruchthülsen- und/oder Samenmehle Walnuß-, Haselnuß-, Mandelschalenmehl, Oliven-, Aprikosen-, Pfirsich- oder Kirschkernmehl, Mehle von Palmkernen und Kokosnüssen, Jojobafrüchten, Macademia-Nüssen, Pistazien- und Pinienschalen, Maiskolbenmehl, Weizenkleie, Hafermehl oder Holzmehle sowie beliebige Gemische derselben mit einer Korngröße von 50 bis 2000 µm verwendet werden.

8. Verfahren nach den Ansprüchen 1 bis 7, **dadurch gekennzeichnet, daß** als Bleichmittel oxidierende Bleichmittel verwendet werden.

9. Verfahren nach den Ansprüchen 1 bis 8, **dadurch gekennzeichnet, daß** das Bleichmittel in der wäßrigen Suspension eine Konzentration von 1,0 bis 10,0 Gew.-% besitzt.

10. Verfahren nach den Ansprüchen 1 bis 9 **dadurch gekennzeichnet, daß** das Bleichmittel eine wässrige Wasserstoffperoxidlösung ist.

11. Verfahren nach den Ansprüchen 1 bis 10, **dadurch gekennzeichnet, daß** die Endtemperatur der natürlichen Kern-, Schalen-, Fruchthülsen und/oder Samenmehl-Suspension nach der Bleichmittelbehandlung im wesentlichen 15 bis 20°C über der Starttemperatur der Bleichbehandlung liegt.

12. Verfahren nach den Ansprüchen 1 bis 11, **dadurch gekennzeichnet, daß** das behandelte Mehl, abgetrennt, gewaschen und bis zu einer Restfeuchte von 8,0 Gew.-% getrocknet wird.

13. Verfahren nach den Ansprüchen 1 bis 12, **dadurch gekennzeichnet, daß** das behandelte Mehl einen L*-Wert von mindestens 79,5 aufweist.

## Claims

1. Method for producing an abrasive substance, where natural pips, shells, fruit peels and/or seeds are ground to a flour of defined grain size, the flour is treated in an aqueous suspension with 1.0 to 10.0% by weight of a bleaching agent, based on the total batch amount, **characterized in that** the addition of the bleaching agent takes place in two steps, where, in the first step, following the addition of from 20 to 40% by weight of the bleaching agent, based on the total amount, a pH range from 3 to 5 is obtained and the addition of from 60 to 80% by weight of the bleaching agent takes place in the second step together continuously with the addition of an alkali solution in the pH range from 5 to 11.

2. Method according to Claim 1, **characterized in that** the combined continuous addition of bleaching agent and the alkali solution to the suspension is interrupted upon reaching a pH of at most 11 and is resumed after the pH has dropped to 8.0.

3. Method according to Claim 2, **characterized in that** the interruption in the combined continuous addition of bleaching agent and alkali solution is achieved by interrupting the addition of alkali solution.

4. Method according to Claims 1 to 3, **characterized in that** upon reaching a pH of at most 11, this maximum pH is maintained at this value for 5 to 25 minutes.

5. Method according to Claims 1 to 4, **characterized in that** after the combined addition of bleaching agent and alkali solution has been resumed, the addition takes place in such a way that a pH of 8.0 is maintained until the end of the combined addition within a variation range of ± 0.2.

6. Method according to Claims 1 to 5, **characterized in that** upon consumption of the amount of bleaching agent used, the addition of the amount of alkali is also stopped.

7. Method according to Claims 1 to 6, **characterized in that** the natural pip, shell, fruit peel and/or seed flours used are walnut, hazelnut, almond shell flour, olive pip flour, apricot kernel flour, peach kernel flour or cherry pip flour, flours from palm kernels and coconuts, jojoba fruits, macadamia nuts, pistachio and pine shells, corncob meal, wheat bran, oatmeal or wood flours and also any desired mixtures thereof with a grain size of from 50 to 2000 µm.

8. Method according to Claims 1 to 7, **characterized in that** oxidizing bleaching agents are used as bleaching agents.

9. Method according to Claims 1 to 8, **characterized in that** the bleaching agent has a concentration of from 1.0 to 10.0% by weight in the aqueous suspension.

10. Method according to Claims 1 to 9, **characterized in that** the bleaching agent is an aqueous hydrogen peroxide solution.

11. Method according to Claims 1 to 10, **characterized in that** the end temperature of the natural pip, shell, fruit peel and/or seed flour suspension after the treatment with bleaching agent is essentially 15 to 20°C above the starting temperature of the bleaching treatment.

12. Method according to Claims 1 to 11, **characterized in that** the treated flour is separated off, washed and dried to a residual moisture of 8.0% by weight.

13. Method according to Claims 1 to 12, **characterized in that** the treated flour has an L* value of at least 79.5.

## Revendications

1. Procédé pour la préparation d'une substance abrasive, dans lequel on broie des noyaux naturels, des coques, cosses et/ou graines naturelles, en une farine à taille de grain définie, on traite la farine, en suspension aqueuse, par 1,0 à 10,0 % en poids d'un agent de blanchiment, par rapport à la quantité totale de la charge initiale, **caractérisé en ce que** l'addition de l'agent de blanchiment s'effectue en deux étapes, dans une première étape un intervalle de pH de 3 à 5 étant obtenu par addition de 20 à 40 % en poids de l'agent de blanchiment, par rapport à la quantité totale, et dans la seconde étape l'addition de 60 à 80 % en poids de l'agent de blanchiment s'effectuant en continu conjointement avec l'addition d'une solution alcaline dans l'intervalle de pH de 5 à 11.

2. Procédé selon la revendication 1, **caractérisé en ce que** l'addition conjointe continue d'agent de blanchiment et de la solution alcaline à la suspension est interrompue lorsque est atteint un pH de 11 au maximum et est reprise après abaissement du pH à pH 8,0.

3. Procédé selon la revendication 2, **caractérisé en ce que** l'interruption de l'addition conjointe continue d'agent de blanchiment et de solution alcaline est réalisée par interruption de l'addition de la solution alcaline.

4. Procédé selon les revendications 1 à 3, **caractérisé en ce que** lorsqu'un pH de 11 au maximum est atteint, ce pH maximum est maintenu à cette valeur pendant 5 à 25 minutes.

5. Procédé selon les revendications 1 à 4, **caractérisé en ce qu'**après reprise de l'addition conjointe d'agent de blanchiment et de solution alcaline, l'addition s'effectue de manière à maintenir un pH de 8,0, dans les limites de variation de ± 0,2, jusqu'à la fin de l'addition conjointe.

6. Procédé selon les revendications 1 à 5, **caractérisé en ce que** lorsque est consommée la quantité utilisée d'agent de blanchiment, l'addition de la quantité de solution alcaline est également terminée.

7. Procédé selon les revendications 1 à 6, **caractérisé en ce qu'**on utilise comme farines naturelles de noyaux, de coques, de cosses et/ou de graines de la farine de coques de noix, de noisettes, d'amandes, de la farine de noyaux d'olives, d'abricots, de pêches ou de cerises, des farines de coeurs de palmiste et de noix de coco, des fruits de jojoba, des noix de macadamia, des cosses de pistache et de pin, de la farine d'épi de maïs, du son de blé, de la farine d'avoine ou des sciures de bois ainsi que des mélanges quelconques de ceux-ci, ayant une taille de grain de 50 à 2 000 µm.

8. Procédé selon les revendications 1 à 7, **caractérisé en ce qu'**on utilise comme agent de blanchiment des agents de blanchiment oxydants.

9. Procédé selon les revendications 1 à 8, **caractérisé en ce que** l'agent de blanchiment a dans la suspension aqueuse une concentration de 1,0 à 10,0 % en poids.

10. Procédé selon les revendications 1 à 9, **caractérisé en ce que** l'agent de blanchiment est une solution aqueuse de peroxyde d'hydrogène.

11. Procédé selon les revendications 1 à 10, **caractérisé en ce que** la température finale de la suspension de farines naturelles de noyaux, de coques, de cosses et/ou de graines après le traitement par l'agent de blanchiment est pratiquement de 15 à 20 °C supérieure à la température de départ du traitement de blanchiment.

12. Procédé selon les revendications 1 à 11, **caractérisé en ce que** la farine traitée est séparée, lavée et séchée jusqu'à une humidité résiduelle de 8,0 % en poids.

13. Procédé selon les revendications 1 à 12, **caractérisé en ce que** la farine traitée présente une valeur L* d'au moins 79,5.
